# EUROPEAN PATENT SPECIFICATION

(11) **EP 1 946 774 B1**
(45) Date of publication and mention of the grant of the patent: **04.12.2013**
(21) Application number: 06811724.1
(22) Date of filing: 13.10.2006
(51) Int. Cl.: A61K 39/395, A61P 9/10, A61P 25/00, C07K 16/24

(54) **CEREBRAL INFARCTION-PREVENTIVE AGENT**
MITTEL ZUR VERHINDERUNG VON GEHIRNINFARKT
AGENT DE PREVENTION DE L'INFARCTUS CEREBRAL

(30) Priority: 24.10.2005 JP 2005308949
(43) Date of publication of application: 23.07.2008
(73) Proprietor: National University Corporation Okayama University, Okayama-shi, Okayama 700-8530 (JP); Ehime University, Matsuyama-shi, Ehime 790-8577 (JP)
(72) Inventor: NISHIBORI, Masahiro, Okayama-shi Okayama 700-8558 (JP); MORI, Shuji, Okayama-shi Okayama 700-8271 (JP); TAKAHASHI, Hideo, Okayama-shi Okayama 700-8558 (JP); TOMONO, Yasuko, Okayama-shi Okayama 703-8264 (JP); ADACHI, Naoto, Kyoto-shi Kyoto 600-8357 (JP); LIU, Keyue, Ehime 791-0295 (JP)
(74) Representative: Gille Hrabal
(86) International application number: PCT/JP2006/320436
(87) International publication number: WO 2007/049468

(56) References cited:
- WO-A-02/092004
- WO-A2-2006/124477
- JP-A- 2003 520 763
- JP-A- 2005 512 507
- YANG H ET AL: "The cytokine activity of HMGB1" JOURNAL OF LEUKOCYTE BIOLOGY, FEDERATION OF AMERICAN SOCIETIES FOR EXPERIMENTAL BIOLOGY, US, vol. 78, no. 1, 1 July 2005 (2005-07-01), pages 1-8, XP003011643 ISSN: 0741-5400
- LIU KEYUE ET AL: "Anti-high mobility group box 1 monoclonal antibody ameliorates brain infarction induced by transient ischemia in rats." THE FASEB JOURNAL : OFFICIAL PUBLICATION OF THE FEDERATION OF AMERICAN SOCIETIES FOR EXPERIMENTAL BIOLOGY DEC 2007, vol. 21, no. 14, December 2007 (2007-12), pages 3904-3916, XP002556545 ISSN: 1530-6860
- YANG H. ET AL.: 'The cytokine activity of HMGB1' JOURNAL OF LEUKOCYTE BIOLOGY vol. 78, no. 1, July 2005, pages 1 - 8, XP003011643
- O'CONNOR K.A. ET AL.: 'Further characterization of high mobility group box 1 (HMGB1) as a proinflammatory cytokine: central nervous system effects' CYTOKINE vol. 24, 2003, pages 254 - 265, XP003011644
- VILA N. ET AL.: 'Proinflammatory cytokines and early neurological worsening in ischemic stroke' STROKE vol. 31, no. 10, 2000, pages 2325 - 2329, XP003011645
- LIAO S.L. ET AL.: 'Association of immune responses and ischemic brain infection in rat' NEUROREPORT vol. 12, no. 9, 1947, pages 1943 - 1947, XP003011646
- SUGHRUE M E ET AL: "Anti-adhesion molecule strategies as potential neuroprotective agents in cerebral ischemia: a critical review of the literature.", INFLAMMATION RESEARCH : OFFICIAL JOURNAL OF THE EUROPEAN HISTAMINE RESEARCH SOCIETY ... [ET AL.] OCT 2004 LNKD- PUBMED:15597143, vol. 53, no. 10, October 2004 (2004-10), pages 497-508, ISSN: 1023-3830

## Description

### TECHNICAL FIELD

The present invention relates to a drug for suppressing cerebral infarction caused by cerebral ischemia.

### BACKGROUND ART

Cerebral infarction is a disease wherein the brain tissue with impaired blood flow is necrotized due to insufficient blood flow. The insufficient blood flow is caused by the cerebral blood vessel occluded or narrowed due to various factors, such as atherosclerosis of the cerebral blood vessel and transfer of thrombus formed in an extracerebral blood vessel into the brain. Once cerebral infarction occurs, the brain tissue in which necrosis has developed does not regain its function. Therefore, even if the patient survives, symptoms of dementia, motor weakness, sensory abnormality and language disorder often persist. Meanwhile, in recent years, diseases called lifestyle-related diseases, such as hypertension, cardiac diseases, hyperlipidemia and diabetes, are increasing, and thereby the risks for cerebral infarction are increasing. In our country Japan, brain blood vessel diseases mainly including cerebral infarction occupies the third place of cause of death next to cancer and ischemic cardiac disease, as is in European and American advanced countries. Therefore, an effective method for treating cerebral infarction has been earnestly desired.

However, a truly effective treatment has not been found yet until now. For example, a thrombolytic agent such as tissue plasminogen activator is used in order to recover blood flow by removing a thrombus that provokes cerebral infarction. However, since disorders by free radicals generated after recovery of blood flow are also closely related to cerebral damage, the thrombolytic agent alone cannot provide the fundamental solution to treat necrosis of the brain tissue.

In Japan, as a therapeutic for cerebral infarction, only edaravone is approved. However, the agent has side effects such as hepatic and renal damage. In particular, there is data showing that 21.4% of patients who received the agent displayed abnormal values in laboratory tests on the liver function. Even though cerebral infarction may be life-threatening, the treatment with such a high incidence of side effects is problematic. In addition, the drug is a free radical scavenger for protecting a brain tissue from a free radical which is generated during ischemic reperfusion and becomes a cause for a brain tissue disorder. Therefore, it is considered that, although a brain tissue disorder resulted from free radical can be alleviated by edaravone, no effect is exhibited for a brain tissue disorder resulted from other cause such as hypoxic state.

For example, in cerebral infarction, permeability of a brain blood vessel is excessively increased and a protein in blood is leaked out to the outside of a blood vessel to cause cerebral edema. As a mechanism therefor, it is postulated that activity of an enzyme called matrix metalloprotease is excessively heightened and a basement membrane of a blood vessel is digested. Therefore, it is considered that edaravone which is a free radical scavenger can not prevent such a symptom.

In addition, although application of glutamic acid receptor antagonists, estrogen receptor-associated drugs and a matrix metalloprotease inhibitors are studied, it is not considered that the effect of them is sufficient.

Hypothermic therapy is one of the treatments besides medication. However, in addition to high costs required for the operation, infection resulting from lowered immunity and bleeding tendency make application of the treatment difficult generally.

HMGB1, i.e. High Mobility Group box 1, is a protein in which 95% or more of amino acid sequence is equal from a rodent to a human. The HMGB1 is present in a normal cell. However, the blood concentration thereof is increased by stimulation with LPS (liposaccharide) which is an endotoxin released in sepsis, one of systemic inflammatory response syndromes, leading to final tissue failure (e.g. WO 02092004). Therefore, in the technology described in Published Japanese Translation of PCT International Publication No. 2003-520763, an HMG antagonist is administered in order to treat a symptom having a characteristic of activation of an inflammatory cytokine cascade. In addition, an ischemia-reperfusion injury is listed as one example of this symptom, and an antibody that binds to the HMGB1 protein is exemplified as an HMG antagonist.

However, the ischemia-reperfusion injury described in the publication No. 2003-520763 is only one of about 100 kinds of diseases exemplified as a symptom or a disease included in systemic inflammatory response syndrome, and there is no description on the indivisual organs of ischemia-reperfusion injury. Further, in Example of the publication, it is shown that HMGB1 is induced by stimulation with TNF and LPS, a mortality of a mouse due to LPS is reduced by administration of an anti-HMBG1 antibody, and that serum HMBG1 levels are increased in human with sepsis or multiorgan failure (MOF), but it is not specifically demonstrated that the anti-HMGB1 antibody exhibits the effect to treat ischemia-reperfusion injury. At least, a subject of the technology of the publication No. 2003-520763 is treatment of systemic inflammatory disease, and the suppression of cerebral infarction by the technology is not suggested.

Further, it is described in Published Japanese Translation of PCT International Publication No. 2005-512507 that the anti-HMGB1 antibody may treat a disease associated with an inflammatory cytokine cascade, and cerebral infarction is also exemplified as one of the disease.

However, in the publication No. 2005-512507, cerebral infarction is only one example among more than 100 exemplified diseases. Further, diseases for which the therapeutic effect is demonstrated in Example are only puncture of caecum and sepsis, and the effect on cerebral infarction is not demonstrated.

### DISCLOSURE OF THE INVENTION

As described above, drugs for treating or preventing cerebral infarction have previously been known. However, a drug having a novel mechanism of action is desired, since the conventional drugs have problems of adverse side effects and limited efficacy. For example, edaravone which is a cerebral infarction therapeutic being solely approved in Japan has many problems such as adverse side effects, and the efficacy remains a limited level. Therefore, it is considered that truly excellent cerebral infarction suppressant is desired.

Then, an objective to be solved by the present invention is to provide a suppressant for cerebral infarction occurred after long-term ischemia corresponding to actual cerebral infarction, and has little adverse side effect.

In order to solve the above-described problem, the present inventors continued to variously study a drug effective in inhibiting cerebral infarction. As a result, the present inventors found out that an anti-HMGB1 monoclonal antibody has the more excellent effect than any drug which had been reported in the past, resulting in completion of the present invention.

For example, edaravone removes a free radical generated by ischemia-perfusion, and inhibits mainly an ischemia-perfusion injury. By comparison with edaravone, the drug of the present invention mainly exerts direct protective action on ischemic brain tissue, and can suppress brain damage itself due to the hypoxic state and the like. As a result, the drug of the present invention can suppress not only a disorder region due to the hypoxic state, but also an ischemia-perfusion injury formed in vicinity of the region.

A cerebral infarction suppressant of the present invention is characterized in comprising an anti-HMGB1 monoclonal antibody as an active ingredient.

In the present invention, the anti-HMGB1 monoclonal antibody is used for producing a cerebral infarction suppressant.

A method for suppressing cerebral infarction of the present invention is characterized in comprising administering the anti-HMGB1 monoclonal antibody.

The cerebral infarction suppressant of the present invention can effectively suppress brain tissue necrosis resulted from a long term ischemia due to cerebral thrombosis and cerebral embolism or the like. In addition, it is considered that a possibility generating a serious adverse side effect is extremely low based on the relatively safe clinical application of antibody drugs currently used. Therefore, the cerebral infarction suppressant of the present invention is extremely useful as being capable of suppressing the cerebral infarction for which a particularly effective treating means has not been present.

### BRIEF DESCRIPTION OF THE DRAWINGS

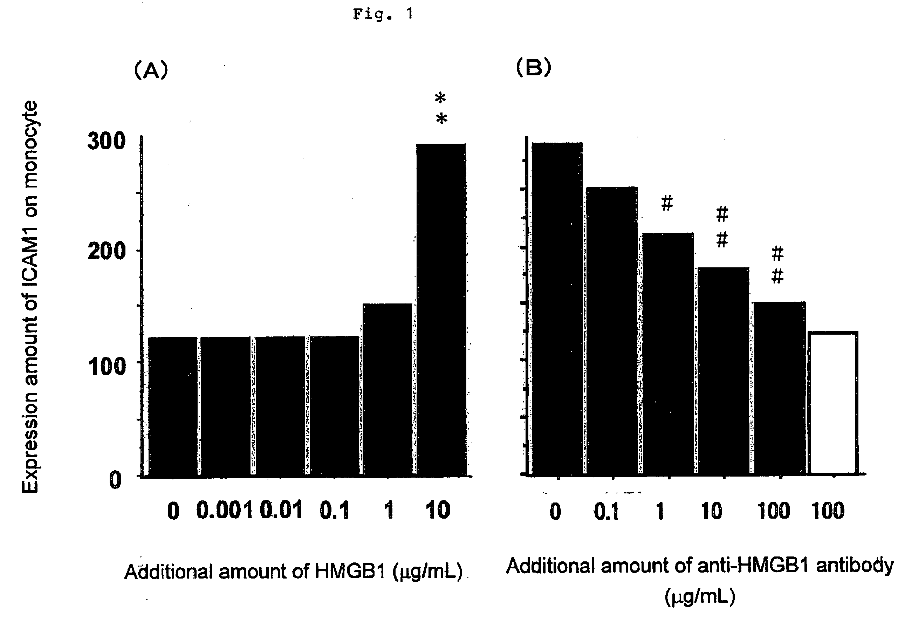

Figure 1 shows the effects of the anti-HMGB1 monoclonal antibody with neutralizing the activity of HMGB1. Figure 1 (A) shows the levels of ICAM-1 expression on monocytes by addition of HMGB1, and Figure 1 (B) shows the levels of ICAM-1 expression in case that the anti-HMGB1 monoclonal antibody is added simultaneously with addition of HMGB1.

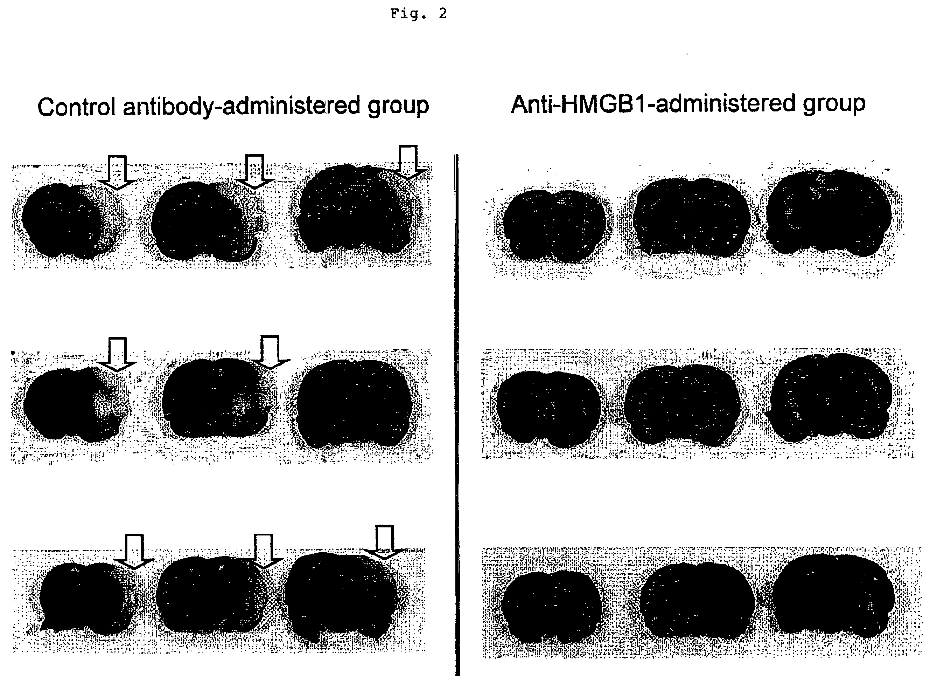

Figure 2 shows the suppressing effects of administration of the anti-HMGB1 monoclonal antibody on cerebral infarction. Twenty four hours after cerebral ischemia for 2 hours, cerebral infarction is generated at a place shown by arrows in a control antibody-administered group. On the other hand, no cerebral infarction is observed in an anti-HMGB1 monoclonal antibody-administered group.

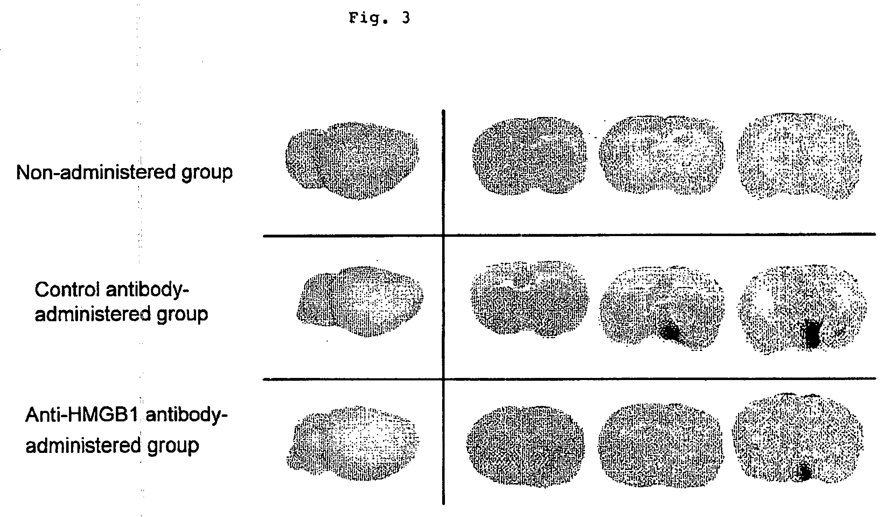

Figure 3 shows the suppressing effects of administration of the anti-HMGB1 monoclonal antibody on the increased permeability of brain blood vessel. In non-ischemic group without load of cerebral ischemia, leakage of Evans blue, i.e. dye, is not seen. On the other hand, in a control antibody-administered group to which cerebral ischemia was loaded and a control antibody was administered, a considerable amount of leakage is observed. In an anti-HMGB1 antibody-administered group to which the anti-HMGB1 monoclonal antibody of the present invention was administered, such a leakage is clearly inhibited.

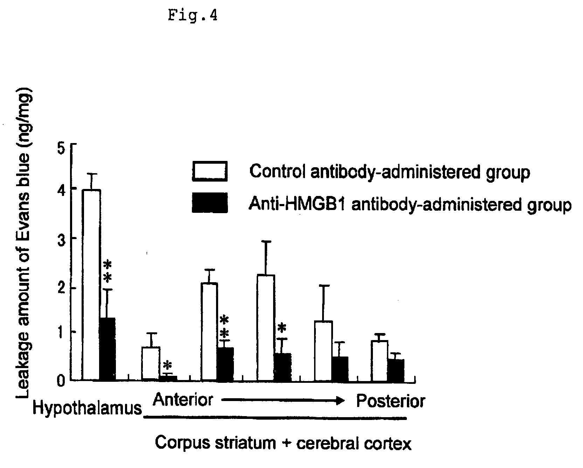

Figure 4 summarizes the quantitative comparison of the leakage of Evans blue into brain tissue between the control antibody-administered group and the anti-HMGB1 antibody-administered group. It is found that the increased permeability of brain blood vessels can be significantly inhibited by administering the aniti-HMGB1 monoclonal antibody.

### BEST MODE FOR CARRYING OUT THE INVENTION

The cerebral infarction suppressant of the present invention contains the anti-HMGB1 monoclonal antibody as one of active ingredients. The anti-HMGB1 monoclonal antibody specifically binds to HMGB1 and neutralizes HMGB1, and inhibits necrosis of brain nerve cells. On the other hand, the antibody does not act on other substances. Therefore, it is considered that there is no or extremely little possibility of production of an adverse side effect.

The anti-HMGB1 monoclonal antibody may be prepared according to a conventional method. For example, a mouse, a rat or the like is immunized using commercially available HMGB1, and its antibody-producing cell or spleen cell and a myeloma cell are fused to obtain a hybridoma. The hybridoma is cloned, and a clone producing an antibody which specifically reacts with HMGB1 is screened. The clone is cultured, and a secreted monoclonal antibody may be purified.

A dosage form and an administration form of the cerebral infarction suppressant of the present invention are not particularly limited, but it is preferable that the suppressant is formulated into an injectable solution and administered intravenously in view of emergency for cerebral ischemia. In that case, as a solvent, an isotonic solution for plasma such as a physiological saline having an adjusted pH and an aqueous glucose solution can be used. Alternatively, when the antibody is lyophilized with a salt, pure water, distilled water, sterile water or the like can be used. A concentration thereof may be that of a normal antibody preparation, and may be around 1 to 5 mg/ml, provided that an osmotic pressure of the injectable solution should be equivalent to that of plasma.

Generally, cerebral infarction is generated by ischemia due to cerebral thrombosis, cerebral embolism or the like, and is accompanied with a morphological lesion, i.e. necrosis, having such an extent of a size that can be seen visually. On the other hand, in apoptosis resulted from a cause of ischemia for an extremely short time such as a few minutes to ten and a few minutes due to transient reduction in a brain blood stream or a small thrombus, omission of only a nerve cell occurs at site which is vulnerable to ischemic insult after a few days. Even when a symptom occurs by the apoptosis, the symptom is considerably milder than the case of cerebral infarction, not leading to life threatening. The cerebral infarction suppressant of the present invention targets cerebral infarction due to long-term ischemia, but it is presumed that the suppressant is also effective for the slow nerve cell apoptosis.

"Long-term" in long-term ischemia is not particularly limited, but refers to at least a time to such an extent that necrosis of a brain tissue is directly caused by ischemia. Examples of the time depends on a cause, an extent of ischemia, an individual difference or the like, but include 1 hour or longer, further 1.5 hours or longer, particularly 2 hours or longer by considering a time from occurrence of ischemia to actual treatment.

The cerebral infarction suppressant according to the present invention is preferably administered during ischemia or after ischemia, though the suppressant may be administered in a preventive manner before ischemia. Namely, the cerebral infarction suppressant of the present invention is administered after the incidence of long-time ischemia solely or concomitantly with the administration of a thrombolytic drug or after reperfusion.

Although the optimum time point to administer the cerebral infarction suppressant of the present invention is not particularly limited, the suppressant is preferably administered during ischemia or after ischemia-reperfusion. There is not a clear distinction between "during ischemia" and "after ischemia-reperfusion" in "during ischemia or after ischemia-reperfusion", and "during ischemia or after ischemia-reperfusion" refers to, for example, just before and just after a certain treatment for ischemia-reperfusion, such as administration of a thrombolytic drug, simultaneously with the treatment or predetermined time after the treatment. At least, administration before ischemia is not included in "during ischemia or after ischemia-reperfusion". When the suppressant is administered before ischemia, the suppressant is substantially deemed to be a preventive agent, and administration before ischemia is difficult in the case of cerebral infarction due to its sudden and unexpected onset.

By administering the cerebral infarction suppressant of the invention during ischemia or after ischemia-reperfusion, it is considered that reduction in brain blood stream at reperfusion, release of glutamate and production of active oxygen which are cause of a reperfusion injury, adhesion of leukocyte to blood vessel endothelium, activation of nerve ending potential-dependent calcium channel or the like can be inhibited, and an extremely initial stage of intracerebral inflammation caused by cerebral ischemia can be inhibited. More preferably, the suppressant is administered immediately after reperfusion. Herein, "immediately after" does not strictly refer to immediately after reperfusion, but refers to within 30 minutes from any treatment for ischemia-reperfusion such as administration of a thrombolytic agent.

As shown in the Examples described later, prominent suppressing effects on cerebral infarction were observed in the case where 200 µg of the anti-HMGB1 monoclonal antibody was administered. From the result, dose of the anti-HMGB1 monoclonal antibody for humans is estimated to be 0.2 to 5 mg/kg, preferably 0.2 to 2 mg/kg per dosage. However, the dose of the suppressant should be suitably changed depending on patient's age, sex and severity of illness and the like.

Additionally, the cerebral infarction suppressant of the present invention is preferably administered several times or in a continuous manner. This is because, in treatment of cerebral infarction, a concentration of the anti-HMGB1 monoclonal antibody in a brain tissue needs to be maintained at a constant concentration or higher over a long period time. Specifically, the suppressant is administered preferably during ischemia-reperfusion or immediately after ischemia-reperfusion and every 6 to 12 hours thereafter. Further, when the suppressant is administered in a continuous manner, each dose is preferably administered over one to several hours by drip infusion and the like.

### EXAMPLES

The present invention will be explained more specifically by examples below.

### Example 1: Preparation of anti-HMGB1 monoclonal antibody

### (a) Immunization of rat

Into a 2 mL-glass syringe, was taken 1mg/mL of a commercially available mixture of bovine thymus-derived HMGB1 and HMGB2 (manufactured by Wako Pure Chemical Industries, Ltd., code No. 080-070741), and an equivalent volume of a complete Freund's adjuvant was taken into another 2 mL-glass syringe. These syringes were connected with a connecting tube. The mixture and the adjuvant were gradually kneaded through the connecting tube to obtain an emulsion. Each 0.1 mL of the obtained emulsion at a total of 0.2 mL was injected to a rat anesthetized with sevoflurane in a hind limb footpads. After 2 weeks, blood was probatively taken from jugular, and the increase of antibody titer was confirmed. Then, an enlarged iliac lymph node was sterilely taken out 5 weeks after the injection administration. From the two lymph nodes obtained, about 6 × 10⁷ cells could be recovered.

### (b) Cell fusion and cloning

The iliac lymph node cell and mouse myeloma SP2/O-Ag14 (SP2) cell were fused using polyethylene glycol, and the obtained fused cell was seeded on a 96-well microplate. After one week, initial ELISA screening was performed, and positive wells were subjected to secondary screening by Western blotting. Well cells exhibiting positive were transferred to a 24-well microplate, and the cells were increased to about 2 ×10⁵ as the almost confluent state. Then, using 0.5 mL of a freezing medium in which 10% bovine fetal serum and 10% dimethyl sulfoxide were added to a GIT medium, the cells were freezing-stored in liquid nitrogen. The freezing-stored cells were thawed, and then subjected to cloning on a 96-well microplate.

### (c) Purification of antibody

The positive cells were cultured for 2 weeks at a large scale with a rotation culturing device (manufactured by Vivascience) to obtain an antibody fluid having a concentration of 2 to 3 mg/mL. The antibody fluid was kneaded with an affinity gel (manufactured by Invitrogen, MEP-HyperCel) under neutral pH to specifically bind the anti-HMGB1 antibody to the gel. The antibody which specifically bound to the gel was eluted by a glycine-hydrochloric acid buffer at pH of 4. The eluate was concentrated with an ultra filtration devise, and thereafter the antibody was further purified with a Sepharose CL6B gel filtration column of diameter 2 cm × length 97 cm.

### Example 2

The neutralization activity of the anti-HMGB1 monoclonal antibody prepared in Example 1 was tested.

First, 1 × 10⁶/mL peripheral blood mononuclear cells were prepared from peripheral blood of a healthy person by a conventional method, and were cultured for 24 hours in a basal medium for culturing an animal cell containing 10% bovine fetal serum (manufactured by Sigma, RPMI1640). Then, bovine HMGB1 purified from a bovine thymus-derived HMGB1/2 mixture manufactured by Wako Pure Chemical Industries, Ltd. was added to the medium at a concentration of 0.001 to 10 µg/mL to stimulate the monocytes. Twenty four hours after addition of HMGB1, the cells were collected, and an expression amount of ICAM-1 (intercellular adhesion molecule-1) expressed on the monocytes with HMGB1 was quantitated by a fluorescent antibody method (FACS method). Results are shown in Fig. 1 (A). In Fig. 1 (A), "**" indicates the case where there was a significant difference at p<0.01 by t-test as compared with the case of addition of no HMGB1. From the result, it was found that expression of ICAM-1 on monocytes is significantly increased by 10 µg/mL of HMGB1.

Then, in the above procedure, 0 to 100 µg/mL of anti-HMGB1 monoclonal antibody was added at the same time with the addition of 10 µg/mL of HMGB1, and expression levels of ICAM-1 were quantitated using a fluorescent antibody method as described above. Results are shown in Fig. 1(B). In Fig. 1 (B), "#" indicates the case where there was a significant difference at p<0.05 by t-test as compared with the case of addition of no antibody, and "##" indicates the case where there was a significant difference at p<0.01. In addition, a rightmost outline column is the result of the case of addition of no HMGB1. From the result, it was verified that the anti-HMGB1 monoclonal antibody prepared in Example 1 at a concentration of 1 µg/mL or higher could significantly neutralize HMGB1.

### Example 3

Fifteen male Wistar rats weighing about 300 g were divided into an anti-HMGB1 monoclonal antibody-administered group of 7 animals and a control antibody-administered group of 8 animals. These rats were anesthetized with a gas mixture of 2% halothane and 50% laughing gas, and kept under spontaneous breathing. Subsequently, a median incision was made in the neck of the rat placed on its back, and the right common carotid artery was exposed. After an intraperitoneal injection of 100 units of heparin, the root of the right middle cerebral artery was occluded by inserting 4.0 nylon thread coated with silicone into the right internal carotid artery from the bifurcation of the internal and external carotid arteries. The tip of the nylon thread was placed 18 mm from the bifurcation. After suture of the incision of the skin, the rats were allowed to recover from anesthesia. During surgery, an electronic thermometer was inserted into the rectum, and the rectum temperature was maintained at 37.0±0.1°C with a lamp. After recovery from anesthesia, paralysis of the contralateral limb was observed in all rats.

Five minutes before reperfusion of blood flow, the rats were anesthetized again. After opening the skin suture, cerebral blood flow was resumed by removing the nylon thread by 5 mm 2 hours after middle cerebral artery occlusion. To the antibody-administered group, was administered 200 µg of the anti-HMGB1 monoclonal antibody dissolved in 0.2 to 0.4 mL of a phosphate-buffered NaCl solution via a tail vein at reperfusion. Further, the same amount of an antibody was administered after 6 hours. To the control antibody-administered group, was administered the same amount of rat IgG.

Twenty-four hours after recovery of blood flow, sodium pentobarbital was intraperitoneally administered to the rats for anesthesia. Subsequently, the brain was perfused with physiological saline added by heparin, and the rat was decapitated. The brain was quickly dissected, and rinsed in physiological saline. The brain was sliced coronally between the optic chiasm and the caucal edge of the mammillary body at a thickness of 2 mm. These brain slices were subjected to incubation in 2% triphenyltetrazolium chloride in phosphate buffer (0.1 mol/L, pH 7.4) at 37°C for 30 minutes. As a result, triphenyltetrazolium chloride was reduced due to the action of dehydrogenase present in viable cells, and the tissue was stained in dark red. On the other hand, the dead tissue in the infarcted area was not stained. These brain slices were preserved in phosphate-buffered formalin overnight. Results of staining of coronary brain sections are shown in Fig. 2. In Fig. 2, a left side is a typical example of three animals of control antibody-administered group; a right side is a typical example of three animals of the anti-HMGB1 monoclonal antibody-administered group; three sections aligned in a transverse direction are derived from the same individual; and a left side of three sections is a rostral section. Thereafter, an experimenter who was a third person not involved in the antibody administration experiment measured a size (unit: mm³) of an infarction site in corpus striatum region and a cerebral cortex region using a computer. In addition, a size of the resulting cerebral infarction site was tested with t-test (unpaired). An average of respective values is shown in Table 1.

**[Table 1]**

| | Control antibody-administered group | Anti-HMGB1 antibody-administered group |
|---|---|---|
| Corpus striatum | 15.7±17.3 | 0±0* |
| Cerebral cortex | 57.1±41.5 | 0±0** |
| Total | 72.8±50.2 | 0±0** |

A value (unit: mm³) in Table indicates a size of cerebral infarction (average ± standard deviation), "*" indicates the case where the value was significant at p<0.05 relative to a corresponding control group, and "**" indicates the case where the value is significant at p<0.01.

According to the result, cerebral infarction was recognized over cerebral cortex region to corpus striatum region in the control group. On the other hand, no cerebral infarction was recognized in the antibody-administered group.

As described above, the cerebral infarction suppressant of the present invention can remarkably suppress formation of a "core" which is a direct lesion core due to the hypoxic state or the like by protecting a brain tissue from influence by ischemia. Therefore, it was verified that the cerebral infarction suppressant of the present invention exhibits extremely excellent activity of suppressing cerebral infarction.

### Example 4

Seventeen male Wistar rats were divided into an anti-HMGB1 monoclonal antibody-administered group of 8 animals and a control antibody-administered group of 9 animals, and local cerebral ischemia for 2 hours was loaded by a similar method to Example 3. To the anti-HMGB1 monoclonal antibody-administered group, was administered each 200 µg of the anti-HMGB1 monoclonal antibody at a total of three times of at reperfusion (immediately after blood stream recovery), and 6 hours and 24 hours after blood stream recovery. To the control antibody-administered group, was administered the same amount of rat IgG. Then, after 48 hours from blood stream recovery, brain slices were made. An average of a value of a size of an infarction site in corpus striatum region and cerebral cortex region is shown in Table 2.

**[Table 2]**

| | Control antibody-administered group | Anti-HMGB1 antibody-administered group |
|---|---|---|
| Corpus striatum | 24.8±16.7 | 6.7±14.5* |
| Cerebral cortex | 62.7±40.4 | 9.0±23.3** |
| Total | 87.5±55.9 | 15.7±37.7** |

From the result, it was verified that cerebral infarction can be significantly suppressed by administering the anti-HMGB1 monoclonal antibody even after 48 hours from blood stream recovery. However, some cerebral infarction is generated as compared with those after 24 hours. This is a result of reperfusion injury persisted with time around a core, i.e. a direct injury region due to the hypoxic state or the like.

In the control group, a wider necrotized region developed 48 hours after reperfusion in the penumbra area around the core, since a core had been formed as described in Example 3. To the contrary, penumbra was also inhibited and only a small necrosis lesion was manifested in the anti-HMGB1 monoclonal antibody-administered group. This may be because formation of a core itself was inhibited as described in Example 3.

As described above, it was verified that, according to the cerebral infarction suppressant of the present invention, cerebral infarction can be remarkably suppressed.

### Example 5

Nine male Wistar rats were divided into an anti-HMGB1 monoclonal antibody-administered group of 3 animals, a control antibody-administered group of 3 animals and a non-administered group of 3 animals, and local cerebral ischemia for 2 hours was loaded to the anti-HMGB1 monoclonal antibody-administered group and the control antibody-administered group by a similar method to Example 3. To the anti-HBG1 monoclonal antibody-administered group, was administered 200 µg of the anti-HMGB1 monoclonal antibody immediately after blood stream recovery. Then, immediately after administration of the anti-HMGB1 monoclonal antibody, 2% Evans blue saline was administered at a dose of 20 mg/kg through a tail vein. Since Evans blue is bound to albumin which is a serum protein, albumin leaked out from a blood vessel can be visualized. To the control antibody-administered group, were administered the same amount of an anti-Keyhole Limpet monoclonal antibody and Evans blue. The antibody belongs to the same TgG2a class as that of the anti-HMGB1 monoclonal antibody. In addition, to the non-administered group, only cervical operation was applied, and no local cerebral ischemia was loaded, and only Evans blue was administered as described above.

Three hours after administration of Evans blue and the like, 50 mg/kg pentobarbital was administered intraperitoneally, 150 ml of physiological saline was perfused through a left ventricle under deep anesthesia, and then a brain was isolated. Fig.3 shows a photograph of sections of the isolated brain. In the non-administered group, transfer of Evans blue from a blood vessel to the brain is hardly recognized. In the control antibody-administered group, transfer of Evans blue into a brain is recognized in any of hypothalamus, corpus striatum and cerebral cortex on an ischemia side, indicateing that brain blood vessel permeability of these regions was increased. On the other hand, the permeability of brain blood vessel observed in the control antibody-administered group was remarkably suppressed in the anti-HMGB1 antibody-administered group. Especially, leakage of Evans blue was almost suppressed in corpus striatum and cerebral cortex in which an infarction lesion is formed after 24 hours.

In addition, a leakage amount of Evans blue was quantitated by homogenizing the brain of the anti-HMGB1 antibody-administered group and the control antibody-administered group in a mixed solvent of 0. 6 N H₃PO₄: acetone = 5:13 and by extracting Evans blue. Results are shown in Fig. 4. In Fig. 4, "*" indicates the case where the value was significant at <0. 05 relative to the corresponding control, and "**" indicates the case where the value was significant at p<0.01. From the result, it was verified that the anti-HMGB1 monoclonal antibody can significantly suppress leakage of Evans blue.

From the above results, it is found that, by administering the anti-HMGB1 monoclonal antibody, leakage of a blood protein into brain tissue due to cerebral ischemia can be effectively suppressed, and it is possible to suppress brain edema which is a cause for a brain disorder.

### Comparative Example 1

In order to further demonstrate the excellent suppressing effect of the suppressant of the present invention on cerebral infraction, the cerebral infarction suppressing effect of edaravone, i.e. 3-methyl-1-phenyl-2-pyrazolin-5-one, which is solely approved in Japan as a drug for directly treating cerebral infarction, was tested by a similar method to Example 3.

Specifically, twelve male Wistar rats weighing about 300 g were divided into an edaravone-administered group of 6 animals and a physiological saline-administered group (control group) of 6 animals, and local cerebral ischemia for 2 hours was loaded by a similar method to Example 3. To the edaravone-administered group, was administered 3 mg/kg edaravone through a tail vein at reperfusion. Further, after 6 hours, the same amount of edaravone was administered through a tail vein. In addition, to the control group was administered the same amount of a physiological saline. After 24 hours from blood stream recovery, brain slices were prepared and stained as Example 3. Thereafter, an experimenter who was a third person not involved in the experiment measured a size (unit: mm³) of an infarction site in corpus striatum region and cerebral cortex region using a computer (software: Scion Image). In addition, a size of the estimated cerebral infarction site was tested with t-test (unpaired). Results are shown in Table 3. In Table 3, "NS" indicates that there is no significant difference relative to the control group.

**[Table 3]**

| | Control group | Edalabon-administered group |
|---|---|---|
| Corpus striatum | 45.2±9.0 | 29.1±10.5 NS |
| Cerebral cortex | 77.8±10.8 | 60.2±17.0 NS |
| Total | 123.0±19.0 | 89.3±26.4 NS |

As shown in the result of Table 3, when edaravone is administered after cerebral ischemia-reperfusion, a size of cerebral infraction is reduced by about 22% in cerebral cortex, by about 35% in corpus striatum, and by a total of about 27%, relative to the control group. However, these cerebral infarction suppressing effects of edaravone were not statistically significant in any of cerebral cortex, corpus striatum and a total of these.

On the other hand, as shown in results of Examples 3 and 4, the anti-HMGB1 monoclonal antibody can statistically significantly and potently suppress cerebral infarction in any of cerebral cortex, corpus striatum and a total of them.

Therefore, it was verified that the anti-HMGB1 monoclonal antibody has the more excellent cerebral infarction suppressing effect than edaravone which is approved as a cerebral infarction suppressant.

## Claims

1. A HMGB1 (=High Mobility Group Box 1) neutralizing anti-HMGB1 monoclonal antibody for use as a suppressant in the treatment of cerebral infarction, **characterized in that** the expression of ICAM-1 (=intercellular adhesion molecule-1) on monocytes is inhibited.

2. The anti-HMGB1 monoclonal antibody for use according to claim 1, wherein the cerebral infarction is due to long-term ischemia.

3. The anti-HMGB1 monoclonal antibody for use according to claim 1 or 2, wherein the suppressant is to be administered during ischemia or after ischemia-reperfusion.

4. The anti-HMGB1 monoclonal antibody for use according to any one of claims 1 to 3, wherein the suppressant is to be administered at plural times.

5. The anti-HMGB1 monoclonal antibody for use according to claim 4, wherein the suppressant is to be administered during ischemia or immediately after ischemia-reperfusion, and then every 6 to 12 hours.

6. The anti-HMGB1 monoclonal antibody for use according to claim 4 or 5, wherein the anti-HMGB1 monoclonal antibody is to be administered at 0.2 to 5 mg/kg per one time.

7. The anti-HMGB1 monoclonal antibody for use according to any one of claims 1 to 3, wherein the suppressant is to be continuously administered during ischemia or after ischemia-reperfusion.

## Patentansprüche

1. Ein HMGB1 (= High Mobility Group Box 1) neutralisierender Anti-HMGB1 monoklonaler Antikörper zur Verwendung als Hemmer in der Behandlung eines Hirninfarktes, **dadurch gekennzeichnet, dass** die Expression von ICAM-1 (= interzelluläres Adhäsionsmolekül-1) auf Monozyten inhiblert wird.

2. Der Anti-HMGB1 monoklonale Antikörper zur Verwendung nach Anspruch 1, worin der Hirninfarkt auf eine langfristige Ischämie zurückzuführen ist.

3. Der Anti-HMGB1 monoklonale Antikörper zur Verwendung nach Anspruch 1 oder 2, worin der Hemmer während der Ischämie oder nach der Ischämie-Reperfusion verabreicht wird.

4. Der Anti-HMGB1 monoklonale Antikörper zur Verwendung nach einem der Ansprüche 1 bis 3, worin der Hemmer mehrfach verabreicht wird.

5. Der Anti-HMGB1 monoklonale Antikörper zur Verwendung nach Anspruch 4, worin der Hemmer während der Ischämie oder nach der Ischämie-Reperfusion und dann alle 6 bis 12 Stunden verabreicht wird.

6. Der Anti-HMGB1 monoklonale Antikörper zur Verwendung nach Anspruch 4 oder 5, worin der Anti-HMGB1 monoklonale Antikörper mit 0,2 bits 5 mg/kg pro Anwendung verabreicht wird.

7. Der Anti-HMGB1 monoklonale Antikörper zur Verwendung nach einem der Ansprüche 1 bis 3, worin der Hemmer während der Ischämie oder nach der Ischämie-Reperfusion kontinuierlich verabreicht wird.

## Revendications

1. Anticorps monoclonal d'anti-HMGB1 neutralisant de HMGB1 (= groupe box 1 à haute mobilité) pour l'utilisation en tant qu'un suppresseur dans le traitement d'infarctus cérébral, **caractérisé en ce que** l'expression de ICAM-1 (= molécule d'adhésion intercellulaire -1) sur des monocytes est inhibée.

2. Anticorps monoclonal d'anti-HMGB1 pour l'utilisation selon la revendication 1, dans lequel l'infarctus cérébral est à cause de l'ischémie à long terme.

3. Anticorps monoclonal d'anti-HMGB1 pour l'utilisation selon l'une des revendications 1 ou 2, dans lequel le suppresseur doit être administré pendant l'ischémie ou après la reperfusion d'ischémie.

4. Anticorps monoclonal d'anti-HMGB1 pour l'utilisation selon l'une des revendications 1 à 3, dans lequel le suppresseur doit être administré plusieurs fois.

5. Anticorps monoclonal d'anti-HMGB1 pour l'utilisation selon la revendication 4, dans lequel le suppresseur doit être administré pendant l'ischémie ou immédiatement après la reperfusion d'ischémie et en suite chaque 6 à 12 heures,

6. Anticorps monoclonal d'anti-HMGB1 pour l'utilisation selon l'une des revendications 4 ou 5, dans lequel l'anticorps monoclonal d'anti-HMGB1 doit être à 0,2 à 5 mg/kg par une seule fois.

7. Anticorps monoclonal d'anti-HMGB1 pour l'utilisation selon des revendications 1 à 3, dans lequel le suppresseur doit être administré continuellement pendant l'ischémie ou aprés la reperfusion d'ischémie.
